(19) Europäisches Patentamt
European Patent Office
Office européen des brevets

(11) **EP 3 803 410 B1**

(12) **EUROPEAN PATENT SPECIFICATION**

(45) Date of publication and mention
of the grant of the patent:
**24.01.2024 Bulletin 2024/04**

(21) Application number: **19733963.3**

(22) Date of filing: **06.06.2019**

(51) International Patent Classification (IPC):
*G01N 33/68* (2006.01)      *G01N 33/94* (2006.01)
*G16H 50/20* (2018.01)      *G16H 10/20* (2018.01)

(52) Cooperative Patent Classification (CPC):
**G01N 33/6896; G01N 33/9413; G01N 33/9433;
G16H 10/20; G16H 50/20;** G01N 2800/2821;
G01N 2800/2835

(86) International application number:
**PCT/EP2019/064880**

(87) International publication number:
**WO 2019/234194 (12.12.2019 Gazette 2019/50)**

(54) **METHOD OF PERFORMING DIFFERENTIAL DIAGNOSIS OF NEURODEGENERATIVE DISEASES IN A SUBJECT**

VERFAHREN ZUR DURCHFÜHRUNG EINER DIFFERENZIALDIAGNOSE VON NEURODEGENERATIVEN ERKRANKUNGEN BEI EINER PERSON

MÉTHODE DE RÉALISATION D'UN DIAGNOSTIC DIFFÉRENTIEL DE MALADIES NEURODÉGÉNÉRATIVES CHEZ UN SUJET

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.06.2018 LU 100823**

(43) Date of publication of application:
**14.04.2021 Bulletin 2021/15**

(73) Proprietor: **Alzohis**
**75010 Paris (FR)**

(72) Inventors:
• **VERPILLOT, Romain**
**75019 Paris (FR)**
• **THIRIEZ, Hervé**
**78000 Versailles (FR)**

(74) Representative: **Lecomte & Partners**
**76-78, rue de Merl**
**2146 Luxembourg (LU)**

(56) References cited:
**WO-A1-92/13273      WO-A1-2013/038014**

## Description

### Technical field

The invention is directed to a method for performing a differential diagnosis of neurodegenerative diseases in a subject.

### Background art

The performance of a differential diagnosis allows a clinician to categorize the patient, who has then the opportunity to follow an adequate treatment in order to heal, or at least to improve his/her medical conditions.

The neurodegenerative diseases, which are characterized by the progressive loss of structure or function of neurons, are numerous, as shown by the following list: Alzheimer's disease, mental depression, amyotrophic lateral sclerosis, frontotemporal dementia, Parkinson's disease, progressive supranuclear palsy, Parkinson's disease with dementia, *etc.*

The frontiers between those diseases are sometimes difficult to distinguish, so that the clinician in charge of the follow-up of the patients can hesitate and give an inappropriate treatment to one patient. The diagnosis can also be difficult to establish in the sense that it implies expensive and cumbersome experiments, such as medical imaging and/or lumbar punctures to collect a sample of cerebrospinal (CSF) fluid. On the other hand, medical questionnaires are not sufficient for the clinician to be certain of the diagnosed disease.

A link between morphological and functional changes occurring in the monoaminergic ascending system and the physiopathology of AD has been suggested (Simic G. et al., Neuropathol. Appl. Neurobiol., 2009, 35, 532-554 and Trillo L. et al., Neurosci Biobehav. Rev., 2013, 37, 1363-1379). Noradrenergic neurons in the locus coeruleus (LC) are the main source of noradrenergic inputs to numerous regions throughout the brain. Due to its widespread efferent innervation, the LC projection system plays a pivotal regulatory role in processes such as stress and maintenance of cognitive performances (Sara S. J., Nat. Rev. Neurosci., 2009, 10, 211-223). This leads to the quest of catecholamines detection in order to develop new tools for neurodegenerative disease diagnosis.

WO 2013/038014 A1 discloses an *invitro* method for diagnosing Alzheimer's disease in a subject comprising determining several criteria comprising the age of the subject, a score of said subject to a questionnaire adapted for screening cognitive function, a dopamine concentration in one blood sample of said subject, an adrenaline concentration in one blood sample of said subject, a noradrenaline concentration in one blood sample of said subject.

Mixed dementia is also not very well treated since the patients are often diagnosed with just a single type of dementia. In this case, a physician will base the pharmaceutical decisions/prescriptions on the type of dementia that has been diagnosed, which at the end, will not help the patient to improve his/her living conditions.

### Summary of invention

#### Technical Problem

The invention has for technical problem to alleviate at least one of the drawbacks present in the prior art. More particularly, the invention has for technical problem to avoid expensive and cumbersome experiments to diagnose a subject suffering from a neurodegenerative disease or from a mixed dementia. Said disease can be one of the following: Alzheimer's disease, mental depression, amyotrophic lateral sclerosis, frontotemporal dementia, Parkinson's disease, progressive supranuclear palsy, Parkinson's disease with dementia.

#### Technical solution

The invention is directed to an *in vitro* method for performing a differential diagnosis of neurodegenerative diseases in a subject, according to claim 1.

Further developments of the invention are according to dependent claims 2-9.

In general, the particular embodiments of each object of the invention are also applicable to other objects of the invention. To the extent possible, each object of the invention is combinable with other objects.

#### Advantages of the invention

The invention is particularly interesting in that the differential diagnosis of a patient, when said diagnosis is precise, allows the patient to be adequately followed-up by the clinician. Once the patient has been diagnosed with the correct disease she/he suffers from, she/he can receive the best medical care that is going to help her/him to improve her/his medical conditions. With the differential diagnosis allows for detection of a mixed dementia, it is also an advantage to provide adequate "mixed treatment" for responding more efficacy to such health conditions, especially since it is currently difficult to detect such mixed dementia conditions and to know which disease is implicated when a mixed dementia condition is detected.

The use of blood test allows for the ease of repeatability of the diagnosis, allowing the patient to be followed up, to be diagnosed early (and it is known than an early diagnosis favours the treatment and greatly improve the condition of life of the patient and his/her family).

**Brief description of the drawings**

Figure 1: Scheme of the algorithm designed on training subjects preliminary to model's performance validation.
Figure 2: Distribution of the subjects in the initial panel used in the training model step (to establish the reference tables).
Figure 3: Cloud of points using the global note from the point of view from Alzheimer's disease (AD).
Figure 4: Cloud of points using the global note from the point of view from the control group.

**Description of an embodiment**

The present invention has allowed for the development of an efficient differential diagnosis method.
The diseases that are diagnosed thanks to the method of the present invention are the following neurodegenerative diseases: Alzheimer's disease (AD), mental depression (PSY), amyotrophic lateral sclerosis (ALS), fronto-temporal dementia (FTD), Parkinson's disease (PD), progressive supranuclear palsy (PSP) and Parkinson's disease with dementia (PD+). The diagnosis is able to discriminate which neurodegenerative disease a patient is suffering from. The diagnosis method of the present invention also evidently allows for discriminating healthy patients (CTRL or control).

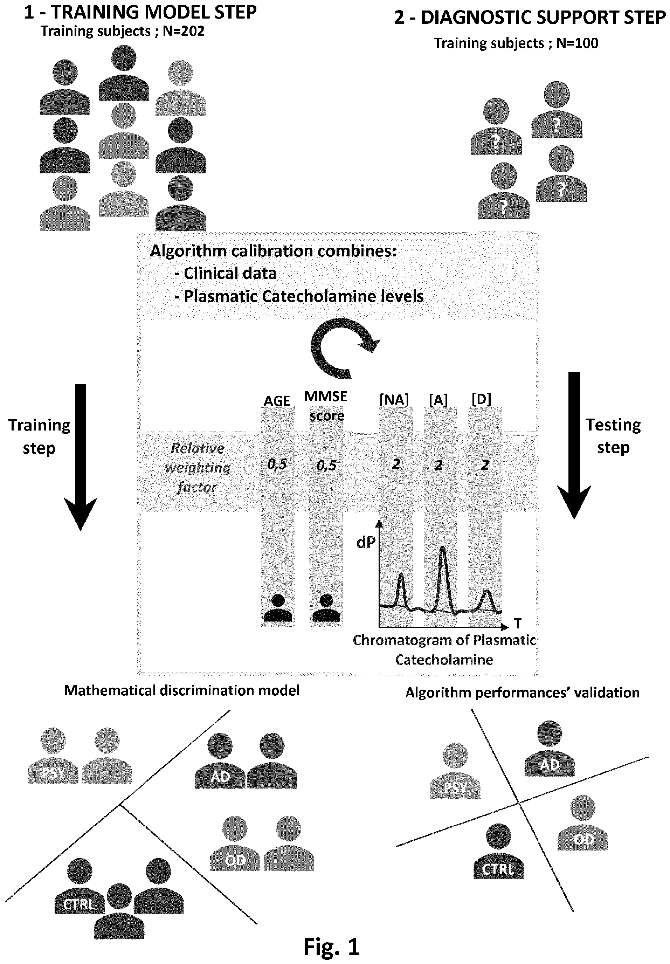

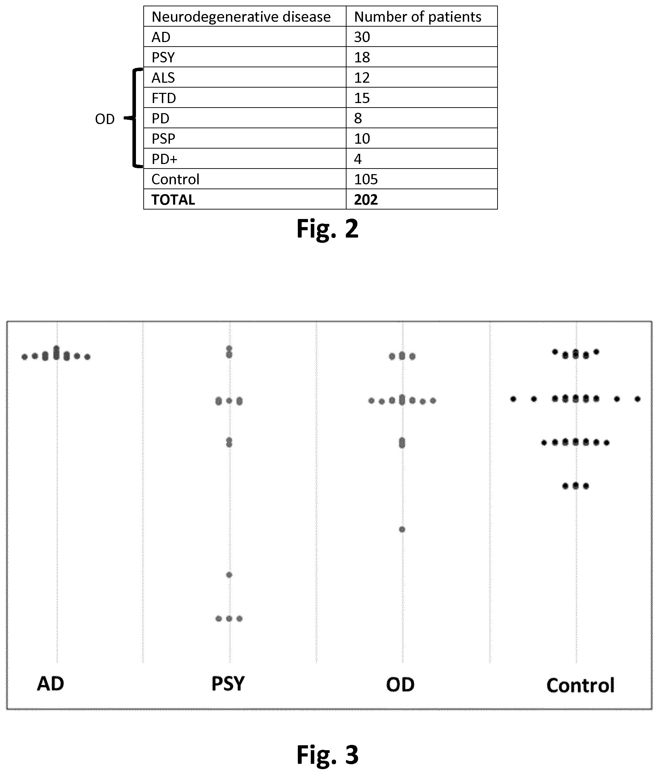

On figure 1, for simplicity, only AD and PSY have been depicted. The rest of the studied diseases has been incorporated under the reference OD (other diseases). This is because the number of subjects in the training model step is less than 15 subjects in these diseases (see also figure 2).
The sensitivity, which is the percentage of patients suffering from the disease and tested as positive among a population of patients identified as suffering from said disease using a reference test), as well as the specificity, which is the percentage of patients who do not suffer from the disease and who were tested as negative among a population of patients identified as not suffering from said disease using a reference test, have been determined for each of the screened neurodegenerative diseases that are diagnosed thanks to the test designed in the present invention. A test with 100% sensitivity will recognize all patients with the disease by testing them positive. A test with 100% specificity will exclude the disease from all healthy patients.
With regard to Alzheimer's disease (AD), the maximum sensitivity is 76.7% and the maximum specificity is 98.3%.
With regard to mental depression (PSY), the maximum sensitivity is 66.7% and the maximum specificity is 92.9%.
With regard to amyotrophic lateral sclerosis (ALS), the maximum sensitivity is 16.7% and the maximum specificity is 97.9%.
With regard to fronto-temporal dementia (FTD), the maximum sensitivity is 33.3% and the maximum specificity is 100%.
With regard to Parkinson's disease (PD), the maximum sensitivity is 75.0% and the maximum specificity is 99.0%.
With regard to progressive supranuclear palsy (PSP), the maximum sensitivity is 40.0% and the maximum specificity is 98.4%.
With regard to Parkinson's disease with dementia (PD+), the maximum sensitivity is 50% and the maximum specificity is 100%.
Finally, with regard to the healthy patients, the maximum sensitivity is 100% and the maximum specificity is 81.4%.
In order to achieve these highly significant statistical results, and to provide therefore a reliable method of differential diagnosis of neurodegenerative disease, two different phases have been achieved. The first phase corresponds to the training model step and the second phase corresponds to the diagnostic support step (see figure 1). In the first phase, a panel of 202 subjects has been tested. Those 202 subjects have been used to determine the reference values on which the diagnosis method of the present invention has been based. As it was known which subjects suffer from which diseases (see figure 2), it was possible to determine the reference values depending on the criteria that are explained below. In the second phase, the diagnosis method has been tested on a smaller panel of 100 subjects in order to confirm the validity of the model.

The following criteria have been used in the present method:

The **age** of the patients that are to be diagnosed is collected, either by asking it clearly to the patients, or by asking it to one of his/her acquaintances, or by checking it in his/her identification papers.

Then, the patients are asked to respond to a medical questionnaire with regard to their cognitive performance. These tests consist of a set of 20 to 30 questions which assess basic cognitive functions. Most assessments require the individual to give basic personal information (*i.e.*, name, address, etc.), to answer simple questions based on common knowledge (*i.e.*, who is the president of the USA?), and to remember simple items of information such as a list of three words or name and address to recall later. According to the response given by the individual, a **score** is given that helps the clinician to categorize the patient. The questionnaires can be one of the abbreviated mental test (AMT), the mini-mental state examination (MMSE) and the six-item cognitive impairment test (6CIT). For the purpose of the present method of differential diagnosis, the MMSE has been chosen, but any other suitable medical questionnaire used to assess basic cognitive functions could be used.

Secondly, a blood sample of each individual is collected and is analysed to determine the level of three catecholamines (CA) into the plasma of the patients. A simultaneous quantitation of epinephrine (**adrenaline**), norepinephrine (**noradrenaline**) and **dopamine** using a standardized HPLC method is performed. The HPLC is coupled with an electrochemical detection. The advantage of this screening is that the collection of blood sample is easy to perform on the individual, especially on the elderly, in comparison to the standard method of diagnosis of neurodegenerative diseases usually requiring the collection of cerebrospinal fluid. It is indeed known that a lumbar puncture is more invasive than blood collection and that lumbar puncture is not always feasible on all kind of patients.

The level of CA into the plasma of the individuals, the results of the MMSE as well as the age of the individuals are taken into account and processed in the following mathematical model.

These first five criteria (age, score to a medical questionnaire, adrenaline concentration ([A]), noradrenaline concentration ([NA]) and dopamine concentration ([D])) are now processed in order to determine which neurodegenerative disease the subject is suffering from.

A parameter based on the catecholamine concentrations can thus be determined in order to refine the diagnosis. It is indeed to be noted that the first five criteria are sufficient to provide the right diagnosis. In the case where a refinement is necessary, the experimentally-determined catecholamine concentrations will be process to determine the *expected concentration* of those catecholamines.

The determination of the expected concentration in the catecholamines is based on the measurement of the disparity between the measured concentration and a concentration of reference. The concentration of reference has been chosen to be the concentration of the catecholamines in the blood sample, more particularly the plasma, in patients suffering from Alzheimer's disease (AD). A mean value of such concentration has been determined among the initial panel of 202 subjects that has been selected in the training model step.

The **adrenaline expected concentration** corresponds to the following mathematical function:

$$[A]^{expected} = \min(K_1; \left| [A] - \overline{[A]_{Alzheimer}} \right|) \quad \textbf{(1a)}$$

meaning that the adrenaline expected concentration corresponds to the minimum value between $K_1$ and the modulus of the difference between the measured adrenaline concentration in the blood sample ([A]) with the mean adrenaline concentration in the blood sample for patients suffering from AD ($\overline{[A]_{Alzheimer}}$),

wherein

$K_1$ is a mathematical constant equal to 15. $K_1$ can be also comprised between 10 and 20 or between 5 and 25. In fact, $K_1$ can be all the integers between 5 and 25, and

$\overline{[A]_{Alzheimer}}$ is the average adrenaline concentration in the blood sample for patients suffering from AD. In fact, $\overline{[A]_{Alzheimer}}$ amounts to 0.3 ng/l.

The **noradrenaline expected concentration** thus corresponds to the following mathematical function:

$$[NA]^{expected} = \min(K_1; \left| [NA] - \overline{[NA]_{Alzheimer}} \right|) \quad \textbf{(1b)}$$

meaning that the noradrenaline expected concentration corresponds to the minimum value between $K_1$ and the modulus of the difference between the measured noradrenaline concentration in the blood sample ([NA]) with the average noradrenaline concentration in the blood sample for patients suffering from AD ($\overline{[NA]_{Alzheimer}}$),

wherein

$K_1$ is a mathematical constant equal to 15. $K_1$ can be also comprised between 10 and 20 or between 5 and 25. In fact, $K_1$ can be all the integers between 5 and 25, and

$\overline{[NA]_{Alzheimer}}$ is the average noradrenaline concentration in the blood sample for patients suffering from AD. In fact, $\overline{[NA]_{Alzheimer}}$ amounts to 0.8 ng/l.

The **dopamine expected concentration** thus corresponds to the following mathematical function:

$$[D]^{expected} = \min(K_1; |[D] - \overline{[D]_{Alzheimer}}|) \qquad \textbf{(1c)}$$

meaning that the dopamine expected concentration corresponds to the minimum value between $K_1$ and the modulus of the difference between the measured dopamine concentration in the blood sample ([D]) with the average dopamine concentration in the blood sample for patients suffering from AD ($\overline{[D]_{Alzheimer}}$),

wherein

$K_1$ is a mathematical constant equal to 15. $K_1$ can be also comprised between 10 and 20 or between 5 and 25. In fact, $K_1$ can be all the integers between 5 and 25, and

$\overline{[D]_{Alzheimer}}$ is the average dopamine concentration in the blood sample for patients suffering from AD. In fact, $\overline{[D]_{Alzheimer}}$ amounts to 0.4 ng/l.

Those values obtained for one subject, either measured (for the age, the score, [A], [NA] and [D]) or determined ($[A]^{expected}$, $[NA]^{expected}$, and $[D]^{expected}$), are compared to a set of reference values. The eight criteria have been measured and/or determined and the following reference tables (Table I and Table II) have been established.

Table I shows the minimum reference values that have been determined in respect to the five criteria (age, score to the medical questionnaire, [NA], [A] and [D]) in relation with the neurodegenerative disease. The minimum for the three calculated criteria used in case of the refinement of the diagnosis method is also indicated ($[NA]^{expected}$, $[A]^{expected}$, and $[D]^{expected}$).

Table I: Reference table showing the lowest value of each criterion in function of the neurodegenerative disease.

|  | Age | MMSE score | [NA] | [A] | [D] | $[NA]^{expected}$ | $[A]^{expected}$ | $[D]^{expected}$ |
|---|---|---|---|---|---|---|---|---|
| AD | 51.1 | 4.0 | 0.06 | 0.15 | 0.12 | 0.01 | 0.00 | 0.01 |
| PSY | 36.8 | 6.0 | 0.12 | 0.35 | 0.12 | 0.35 | 0.08 | 0.06 |
| ALS | 42.8 | 26.0 | 0.32 | 0.37 | 0.32 | 0.18 | 0.00 | 0.05 |
| FTD | 41.2 | 15.0 | 0.22 | 0.28 | 0.24 | 0.13 | 0.06 | 0.11 |
| PD | 29.9 | 26.0 | 0.30 | 0.07 | 0.32 | 0.17 | 0.00 | 0.01 |
| PSP | 45.6 | 25.0 | 0.30 | 0.26 | 0.35 | 0.22 | 0.02 | 0.01 |
| PD+ | 56.1 | 26.0 | 0.32 | 0.17 | 0.12 | 0.41 | 0.02 | 0.13 |
| Control | 40.0 | 28.0 | 0.0 | 0.0 | 0.0 | 0.0 | 0.0 | 0.0 |

Table II shows the maximum reference values that have been determined in respect to the five criteria (age, score to the medical questionnaire, [NA], [A] and [D]) in relation with the neurodegenerative disease. The maximum for the three calculated criteria used in case of the refinement of the diagnosis method is also indicated ($[NA]^{expected}$, $[A]^{expected}$, and $[D]^{expected}$).

Table II: Reference table showing the highest value of each criterion in function of the neurodegenerative disease.

| | Age | MMSE score | [NA] | [A] | [D] | [NA]expected | [A]expected | [D]expected |
|---|---|---|---|---|---|---|---|---|
| AD | 81.5 | 28.0 | 11.4 | 2.6 | 3.5 | 10.0 | 2.0 | 2.9 |
| PSY | 63.3 | 30.0 | 2.6 | 2.7 | 28.2 | 1.8 | 2.1 | 10.0 |
| ALS | 76.6 | 30.0 | 1.6 | 1.1 | 1.2 | 0.8 | 0.5 | 0.6 |
| FTD | 85.6 | 30.0 | 1.9 | 25.0 | 12.3 | 1.1 | 10.0 | 10.0 |
| PD | 78.5 | 29.0 | 114.0 | 0.8 | 10.1 | 10.0 | 0.5 | 9.5 |
| PSP | 78.9 | 27.0 | 8.4 | 0.8 | 0.8 | 7.5 | 0.3 | 0.2 |
| PD+ | 73.0 | 29.0 | 1.6 | 0.6 | 9.4 | 0.8 | 0.4 | 8.8 |
| Control | 100.3 | 30.0 | 3.0 | 0.8 | 0.6 | 2.1 | 0.6 | 0.6 |

Therefore, for subject suffering from Alzheimer's disease (AD), the reference values for the criteria are as follows:

- the age is comprised between 51.1 years and 81.5 years;
- the score at the MMSE is comprised between 4.0 and 28.0 ;
- [NA] is comprised between 0.06 ng/l and 11.4 ng/l ;
- [A] is comprised between 0.15 ng/l and 2.6 ng/l ;
- [D] is comprised between 0.12 ng/l and 3.5 ng/l ;
- [NA]expected is comprised between 0.01 ng/l and 10.0 ng/l ;
- [A]expected is comprised between 0.00 ng/l and 2.0 ng/l ;
- [D]expected is comprised between 0.01 ng/l and 2.9 ng/l.

Said reference values can be in fact written as being comprised between $\mathrm{MINI}_{AD}^{criterion}$ and $\mathrm{MAXI}_{AD}^{criterion}$ .

For subject suffering from mental depression (PSY), the reference values for the criteria are as follows:

- the age is comprised between 36.8 years and 63.3 years;
- the score at the MMSE is comprised between 6.0 and 30.0 ;
- [NA] is comprised between 0.12 ng/l and 2.6 ng/l ;
- [A] is comprised between 0.35 ng/l and 2.7 ng/l ;
- [D] is comprised between 0.12 ng/l and 28.2 ng/l ;
- [NA]expected is comprised between 0.35 ng/l and 1.8 ng/l ;
- [A]expected is comprised between 0.08 ng/l and 2.1 ng/l ;
- [D]expected is comprised between 0.06 ng/l and 10.0 ng/l.

Said reference values can be in fact written as being comprised between $\mathrm{MINI}_{PSY}^{criterion}$ and $\mathrm{MAXI}_{PSY}^{criterion}$ .

For subject suffering from amyotrophic lateral sclerosis (ALS), the reference values for the criteria are as follows:

- the age is comprised between 42.8 years and 76.6 years;
- the score at the MMSE is comprised between 26.0 and 30.0 ;
- [NA] is comprised between 0.32 ng/l and 1.6 ng/l ;
- [A] is comprised between 0.37 ng/l and 1.1 ng/l ;
- [D] is comprised between 0.32 ng/l and 1.2 ng/l ;
- [NA]expected is comprised between 0.18 ng/l and 0.8 ng/l ;
- [A]expected is comprised between 0.00 ng/l and 0.5 ng/l ;
- [D]expected is comprised between 0.05 ng/l and 0.6 ng/l.

Said reference values can be in fact written as being comprised between $\mathrm{MINI}_{ALS}^{criterion}$ and $\mathrm{MAXI}_{ALS}^{criterion}$ .

For subject suffering from frontotemporal dementia (FTD), the reference values for the criteria are as follows:

- the age is comprised between 41.2 years and 85.6 years;

- the score at the MMSE is comprised between 15.0 and 30.0 ;
- [NA] is comprised between 0.22 ng/l and 1.9 ng/l ;
- [A] is comprised between 0.28 ng/l and 25.0 ng/l ;
- [D] is comprised between 0.24 ng/l and 12.3 ng/l ;
- [NA]$^{expected}$ is comprised between 0.13 ng/l and 1.1 ng/l ;
- [A]$^{expected}$ is comprised between 0.06 ng/l and 10.0 ng/l ;
- [D]$^{expected}$ is comprised between 0.11 ng/l and 10.0 ng/l.

Said reference values can be in fact written as being comprised between $\mathrm{MINI}_{FTD}^{criterion}$ and $\mathrm{MAXI}_{FTD}^{criterion}$ .

For subject suffering from Parkinson's disease (PD), the reference values for the criteria are as follows:

- the age is comprised between 29.9 years and 78.5 years;
- the score at the MMSE is comprised between 26.0 and 29.0 ;
- [NA] is comprised between 0.30 ng/l and 114.0 ng/l ;
- [A] is comprised between 0.07 ng/l and 0.8 ng/l ;
- [D] is comprised between 0.32 ng/l and 10.1 ng/l ;
- [NA]$^{expected}$ is comprised between 0.17 ng/l and 10.0 ng/l ;
- [A]$^{expected}$ is comprised between 0.00 ng/l and 0.5 ng/l ;
- [D]$^{expected}$ is comprised between 0.01 ng/l and 9.5 ng/l.

Said reference values can be in fact written as being comprised between $\mathrm{MINI}_{PD}^{criterion}$ and $\mathrm{MAXI}_{PD}^{criterion}$ .

For subject suffering from progressive supranuclear palsy (PSP), the reference values for the criteria are as follows:

- the age is comprised between 45.6 years and 78.9 years;
- the score at the MMSE is comprised between 25.0 and 27.0 ;
- [NA] is comprised between 0.30 ng/l and 8.4 ng/l ;
- [A] is comprised between 0.26 ng/l and 0.8 ng/l ;
- [D] is comprised between 0.35 ng/l and 0.8 ng/l ;
- [NA]$^{expected}$ is comprised between 0.22 ng/l and 7.5 ng/l ;
- [A]$^{expected}$ is comprised between 0.02 ng/l and 0.3 ng/l ;
- [D]$^{expected}$ is comprised between 0.01 ng/l and 0.2 ng/l.

Said reference values can be in fact written as being comprised between $\mathrm{MINI}_{PSP}^{criterion}$ and $\mathrm{MAXI}_{PSP}^{criterion}$ .

For subject suffering from Parkinson's disease with dementia (PD+), the reference values for the criteria are as follows:

- the age is comprised between 56.1 years and 73.0 years;
- the score at the MMSE is comprised between 26.0 and 29.0 ;
- [NA] is comprised between 0.32 ng/l and 1.6 ng/l ;
- [A] is comprised between 0.17 ng/l and 0.6 ng/l ;
- [D] is comprised between 0.12 ng/l and 9.4 ng/l ;
- [NA]$^{expected}$ is comprised between 0.41 ng/l and 0.8 ng/l ;
- [A]$^{expected}$ is comprised between 0.02 ng/l and 0.4 ng/l ;
- [D]$^{expected}$ is comprised between 0.13 ng/l and 8.8 ng/l.

Said reference values can be in fact written as being comprised between $\mathrm{MINI}_{PD+}^{criterion}$ and $\mathrm{MAXI}_{PD+}^{criterion}$ .

For a healthy control, the reference values for the criteria are as follows:

- the age is comprised between 40.0 years and 100.3 years;
- the score at the MMSE is comprised between 28.0 and 30.0 ;
- [NA] is comprised between 0.0 ng/l and 3.0 ng/l ;
- [A] is comprised between 0.0 ng/l and 0.8 ng/l ;
- [D] is comprised between 0.0 ng/l and 0.6 ng/l ;

- [NA]^expected is comprised between 0.0 ng/l and 2.1 ng/l ;
- [A]^expected is comprised between 0.0 ng/l and 0.6 ng/l ;
- [D]^expected is comprised between 0.0 ng/l and 0.6 ng/l.

Said reference values can be in fact written as being comprised between $MINI_{healthy\ control}^{criterion}$ and $MAXI_{healthy\ control}^{criterion}$.

A correction factor $\delta$ can be attributed to these lowest and highest reference values. Said correction factor $\delta$ can be comprised between 0.50 and 1.50. Preferentially, said correction factor $\delta$ can be comprised between 0.75 and 1.25. If the correction factor $\delta$ is equal to 1, then the reference values that are used in the diagnostic method of the present invention are in fact the reference values as shown in Table I and in Table II.

The correction factor $\delta$ is used to take into consideration any possible deviation that can occur during the measurements of the catecholamine concentrations or the possible errors made by inadvertence at the determination of the MMSE score. In fact, for a correction factor $\delta$ different from 1, the values of Table I and Table II slightly differ. Iteration of the method of the present invention on one subject with different reference tables (obtained via different correction factor $\delta$) can provide a better robustness of the results. For instance, if a patient is diagnosed with progressive supranuclear palsy with 3 different reference tables, then the certainty of the diagnosis is increased in comparison if said patient would have been diagnosed as suffering from this specific disease by reference to only 1 reference table.

The reference values, or the corrected reference values, are used to compare the at least five criteria (or even the eight criteria). The comparison step is in fact determinant to express a global note (or a global score) for each neurodegenerative disease. To obtain the global note, which will be used to diagnose the subject, a virtual value is firstly determined for each criterion.

If the subject presents a value for a criterion which is outside the range of the reference values (the lower limit being expressed in Table I, or in a corrected Table I if a correction factor $\delta$ has been applied; the upper limit being expressed in Table II, or in a corrected Table II in case where a correction factor $\delta$ is applied), then the virtual value is set to zero. In other words, the selected criterion does not count.

If the subject presents a value for a criterion which is inside the range of the reference values (as indicated above, in accordance with the reference values of (corrected) Table I or (corrected) Table II), then, the following mathematical equation is applied:

$$V_{disease}^{criterion} = 1 + K \frac{VAL^{criterion} - MINI_{disease}^{criterion}}{MAXI_{disease}^{criterion} - MINI_{disease}^{criterion}} \qquad (2)$$

wherein VAL^criterion is the measured value corresponding to a criterion. For instance, if the virtual value for the criterion of age of the subject is to be determined, VAL represents the actual age of said subject. However, if the virtual value for the level of adrenaline in the blood sample of the subject is to be determined, then VAL is the actual measured level of adrenaline in the blood sample (or in the plasma) of the subject.

$MINI_{disease}^{criterion}$ and $MAXI_{disease}^{criterion}$ represent respectively the lowest and the highest reference values obtained, possibly adjusted with the correction factor $\delta$. In fact, the lowest reference values (without applying the correction factor $\delta$) are those listed in Table I and the highest reference values are those listed in Table II (see also paragraphs below said tables).

K is a mathematical parameter, varying between 0 and 0.1. Preferentially, K is comprised between 0 and 0.05.

In order to determine the global note in relation with each neurodegenerative disease, the virtual value in relation with all of the five criteria (age, score, [A], [NA], [D]) and/or with the three supplementary criteria (([A]^expected, [NA]^expected, and [D]^expected) is to be determined in accordance with the previous mathematical equation. Five or eight virtual values are thus calculated.

A weighting factor is then attributed to each of the virtual values. The virtual values in relation with the age and with the score are less relevant than the virtual values in relation with the concentration or the expected concentration. It is indeed more objective to determine scientifically a concentration of a catecholamine in the blood than asking questions to a person. The age is also a more subjective parameter since its relation with respect to the disease is rather variable and depends on the individuals rather than a determined law. For those reasons, the weighting factor for the virtual values in respect of the age and the score of the medical questionnaire has been set for instance to 0.5, while the weighting factor for the virtual values in respect with the concentrations or the expected concentration has been set for instance to 2.

The global note in relation with each neurodegenerative disease is merely obtained by adding all the weighted virtual values together. The mathematical writing is thus as follows:

$$GLOBAL\ NOTE = w_1 V_{age} + w_1 V_{score} + w_2 \left( V_{[A]} + V_{[NA]} + V_{[D]} \right) \qquad (3)$$
$$+ w_2 \left( V_{[A]expected} + V_{[NA]expected} + V_{[D]expected} \right)$$

wherein $w_1$ is a number comprised between 0.1 and 0.9, preferentially between 0.3 and 0.7. For instance, $w_1$ is equal to 0.5.
wherein $w_2$ is a number comprised between 1.1 and 3.0, preferentially between 1.5 and 2.5. For instance, $w_2$ is equal to 2.

The global note in relation with each neurodegenerative disease obtained for a sample of 10 subjects is indicated in Table III. To make the diagnosis, the clinician compares the values of the obtained global notes for each of the eight neurodegenerative diseases and concludes that the patient suffers from a specific disease if the global note for this specific disease is the highest among the eight global notes. The last two columns of Table III show the conclusion of the diagnosis, the 1st diagnosis being the one which is the most probable for the patient. When the interval between the global notes is too weak, typically when the interval is inferior to 2.00 (preferentially inferior to 1.00, more preferentially inferior to 0.50), then a second diagnosis is made. Such patients, where two diagnosis can be made, or even when a third diagnosis can be made (not shown in Table III), generally suffers from mixed dementia. It has also been determined that patient suffering from mental depression (PSY) also shown signs of Alzheimer's disease (AD), mental depression being precursory signs of Alzheimer's disease (AD).

Table III: Global note in relation with each neurodegenerative disease calculated for 10 subjects and conclusion of the differential diagnosis method of the present invention.

| | AD | PSY | ALS | FTD | PD | PSP | PD+ | Control healthy patient | 1st | 2nd |
|---|---|---|---|---|---|---|---|---|---|---|
| 1 | **40.15** | 35.25 | 35.35 | 30.18 | 35.28 | 35.41 | 35.29 | 35.48 | **AD** | / |
| 2 | **40.45** | 30.25 | 35.60 | 35.38 | 35.38 | 35.59 | 25.45 | 35.54 | **AD** | / |
| 3 | 35.16 | **40.44** | 35.44 | **40.34** | 35.44 | 30.43 | 35.48 | 35.48 | **PSY** | FTD |
| 4 | **40.36** | **40.39** | 30.75 | 30.34 | 25.33 | 15.21 | 25.41 | 30.44 | **PSY** | AD |
| 5 | 35.09 | **40.37** | 35.48 | **40.33** | 35.45 | 30.35 | 30.47 | 35.46 | **PSY** | FTD |
| 6 | **40.13** | **40.21** | 35.33 | 35.15 | 35.27 | 25.22 | 35.24 | 35.47 | **PSY** | AD |
| 7 | **40.29** | **40.37** | 30.44 | 35.31 | 30.31 | 25.32 | 35.32 | 35.47 | **PSY** | AD |
| 8 | 30.13 | **40.57** | 20.38 | 30.23 | 35.47 | 25.27 | 25.39 | 35.64 | **PSY** | / |
| 9 | 35.17 | **40.16** | 30.27 | 35.10 | 35.20 | 30.36 | 30.25 | 30.42 | **PSY** | / |
| 10 | 30.56 | **40.83** | 20.46 | 35.69 | 20.21 | 15.07 | 15.40 | 20.29 | **PSY** | / |

In fact, for each patient, the distance between the score of the patient and the cloud of patients in each category is measured. For instance, when in formula **(2)** the reference values for AD are taken into account (namely the value for $MINI_{disease}^{criterion}$ and the value for $MAXI_{disease}^{criterion}$ are those corresponding to $MINI_{AD}^{criterion}$ and to $MAXI_{AD}^{criterion}$, *i.e.*, for example, in case where the criterion of [NA] is concerned, 0.06 and 11.4, respectively), one representation as the one depicted in figure 3 can be drawn. In this case, all the patients suffering from AD have high and concentrated score. In the other three categories (PSY, OD and Control), there are some subjects who also show scores similar to the scores of the patients in the AD category. But, this does not mean that they would eventually be diagnosed as suffering from AD, because they may have obtained a better score in another category.

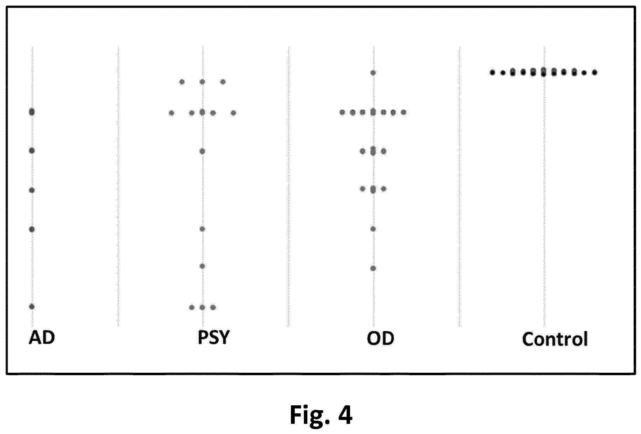

That is the reason why the global note must be calculated by taking into account all the neurodegenerative diseases under study. For instance, when the reference values in formula **(2)** are used for the control group, a representation as the one shown in figure 4 is drawn for the very same group of patient.

By applying this algorithm to each patient, the final suggested diagnosis is the diagnosis of the cloud of points (amongst the 8 possible original clouds) to which the patient is the closest according to the distance measured. If a second-best diagnosis (as shown in Table III) (and possibly a third-best diagnosis) has a close enough score, this second-best, and potentially the third-best, may also be indicated to the clinician.

It is therefore possible for an individual to be diagnosed from a neurodegenerative disease or from mixed dementia. The attribution of a global note for each neurodegenerative disease and for each patient allows the clinician not only to categorize the patient in one class of disease (for instance, by assessing that said patient suffers from AD) but also to give an insight to what other disease this same patient could suffer from (based on the second-best and potentially the third-best diagnosis).

An additional criterion that is used is the determination whether the subject in question suffers from arterial hypertension. It has been indeed noticed that the prevalence of arterial hypertension is four times less in patients suffering from AD. This could be linked to the formation of aggregate in the brain and the low clearance of those aggregates (due to (extremely) low blood pressure in these patients). Therefore, the determination of the blood pressure in the subject can be a further parameter in order to determine whether the subject suffers from AD.

The accurate detection and identification of catecholamines simultaneously in the blood lays a solid foundation for the study of the potential links between AD and catecholamines. In this exploratory study, with a cohort of patients (n = 202) suffering from dementia and other neurodegenerative diseases, we demonstrate the interest of the plasma catecholamine assay for the development of less invasive and accessible solution for the diagnosis of AD, other neurodegenerative diseases, such as PSY, ALS, FTD, PD, PSP and PD+, and also mixed dementia. The statistical exploitation of the signature of catecholamines, coupled with simple data such as age, the mini-mental state examination (MMSE) and the determination of blood pressure, makes it possible to reach for the diagnosis of AD with high sensitivity and specificity performances, namely with 76.8% and 98.2% respectively.

The diagnosis method of the present invention is very useful not only to diagnose a subject, but it is also a tool for providing the follow-up of those subjects. In particular, it is highlighted that the preparation of samples is performed easily, since it is based on a simple blood test. For the medical research point of view, this diagnosis method is also very practical, since, due to the simple detection method used, new treatment and new medicines can be easily assessed.

**Analytical method**

Blood collected in lithium heparin tubes was centrifuged at 3500 rpm for 10 min at +4°C. 1 mL plasma and 100 $\mu$L of 2,3-dihydroxybenzoic acid (DHBA) internal standard (from 100 nM DHBA stock solution) was added to extraction tubes with 100 mg of aluminum oxide previously activated with a mixture of 500 $\mu$L TRIS 3M and 100 $\mu$L EDTA 10 % (5:1) according to a known method (Anton A. H., et al., J. Pharmacol. Exp. Ther., 1962, 138(3), 360-375). Samples were stirred for 10 min and centrifuged at 2000 rpm at 4°C. The aluminum oxide with bound catecholamines was then washed 3 times with 5 mL of distilled water followed by centrifugation. The elution of catecholamines was achieved by the addition of 500 $\mu$L of $HClO_4$ 0.2 N and subsequent centrifugation at 2000 rpm for 10 min at 4°C. Aliquots of 100 $\mu$L were injected into the HPLC system (Waters 515 HPLC Pump, Waters Model 717 autosampler injector), which was equipped with a Purospher® STAR RP-C18 endcapped (5 $\mu$m). The mobile phase consisted of 50 mM sodium acetate buffer containing 0.9 mM sodium lauryl sulfate, 0.3 mM EDTA, 17.5 mM acetic acid and 12 % methanol (vol/vol) at pH 3.6. Electrochemical detection (Coulochem II detector) was performed using an ESA 5010 cell with a glassy carbon working electrode set at a potential of +360 mV vs. Ag/AgCl.

**Description of sample preparation**

The catecholamines (adrenaline, noradrenaline and dopamine) are extracted from the plasma matrix by adsorption on alumina before the HPLC analysis. Sample preparation is simple, because pH-adjustment of the plasma samples is not necessary. Moreover, samples preparation requires only washing steps with Wash Buffer. A selected HPLC column in combination with mobile phase, optimised for this particular separation, allows for sure and reliable chromatographic quantification. With a certified HPLC kit, one person can analyse up to 100 plasma samples per day.

Extraction: Label a sample clean up cartridge appropriately for each sample. Add 0.5 ml extraction buffer to each cartridge and shake briefly. Then add 1 ml plasma (total capacity of the cartridge is 1.5 ml) and 50 $\mu$l Internal Standard (= 600 pg DHBA). Close the cartridge with the top plug and mix for 10 min. Then remove the bottom plug from the cartridge and remove the plasma supernatant by using a vacuum equipment or centrifugation (place the cartridge in a disposable centrifugation tube and centrifuge 1 min at 2000 rpm).

Wash steps: Re-mount the bottom plug and remove the top plug. Add 1 ml Wash Buffer. Close the cartridge again and mix for 30 s (vortex). Then remove the bottom plug from the cartridge and remove the plasma supernatant by using a vacuum equipment or centrifugation (place the cartridge in a disposable centrifugation tube and centrifuge 1 min at 2000 rpm). Repeat this step 2 more times. After the last (third) wash step dry the cartridges well by centrifugation (2 min at 4000 rpm). To ensure that the Wash Buffer is removed completely, tap at the cartridge; the alumina should loosen from the frit. Discard bottom plug.

Elution: Prior to elution attach the plastic tube supplied with the kit to the outlet of the sample clean up column. Add 120 μl Elution Buffer, shake briefly, and wait for 5 min. Then mix for 30 s (vortex). Place the sample clean up column (with the plastic tube attached) in a fresh vial and centrifuge 1 min at 2000 rpm. The eluted sample is collected in the attached tube (This tube can be directly placed into a Waters WISP autosampler).

**Claims**

1. An *in vitro* method for performing a differential diagnosis of neurodegenerative diseases in a subject, said subject being selected among subjects suffering from Alzheimer's disease, mental depression, amyotrophic lateral sclerosis, frontotemporal dementia, Parkinson's disease, progressive supranuclear palsy, and/or Parkinson's disease with dementia,

   said method comprising the steps of:

   a) determining at least five criteria of said subject, said at least five criteria comprising: the age of said subject, a score of said subject to a questionnaire adapted for screening cognitive function, a dopamine concentration [D] in one blood sample of said subject, an adrenaline concentration [A] in one blood sample of said subject, a noradrenaline concentration [NA] in one blood sample of said subject;

   b) determining for each neurodegenerative disease a value $V_{disease}^{criterion}$ per criterion by comparing a measured value VAL$^{criterion}$ corresponding to the obtained criterion in step a) with a range of reference values as follows:

   i. $V_{disease}^{criterion} = 0$ if VAL$^{criterion}$ is outside the range of reference values $MINI_{disease}^{criterion}$ - $MAXI_{disease}^{criterion}$, or

   ii. $V_{disease}^{criterion} = 1 + K \frac{VAL^{criterion} - MINI_{disease}^{criterion}}{MAXI_{disease}^{criterion} - MINI_{disease}^{criterion}}$ if VAL$^{criterion}$ is within the range of reference values

   wherein $MINI_{disease}^{criterion}$ and $MAXI_{disease}^{criterion}$ represent respectively the minimum and the maximum values of the range of reference values with respect to each neurodegenerative disease in function of each criterion,
   wherein K is a mathematical parameter comprised between 0 and 0.1

   c) calculating a global note for each neurodegenerative disease by summing the values $V_{disease}^{criterion}$ each multiplied with a weighting factor;
   d) determining, based on the highest global note calculated in step c) for all of neurodegenerative diseases, whether said subject suffers from Alzheimer's disease, mental depression, amyotrophic lateral sclerosis, frontotemporal dementia, Parkinson's disease, progressive supranuclear palsy, Parkinson's disease with dementia or mixed dementia.

2. The method according to claim 1, wherein in step (a) the at least five criteria further comprises the following criteria:

   - a dopamine expected concentration [D]$^{expected}$ in one blood sample of said subject, said dopamine expected concentration is obtained by applying the following equation

   $$[D]^{expected} = \min\left(K_1; \left|[D] - \overline{[D]_{Alzheimer}}\right|\right)$$

   wherein $K_1$ is a constant comprised between 5 and 25, and
   wherein $[D]_{Alzheimer}$ is 0.4 ng/l.

   - an adrenaline expected concentration [A]$^{expected}$ in one blood sample of said subject said adrenaline expected concentration is obtained by applying the following equation

   $$[A]^{expected} = \min\left(K_1; \left|[A] - \overline{[A]_{Alzheimer}}\right|\right)$$

wherein $K_1$ is a constant comprised between 5 and 25, and
wherein $[A]_{Alzheimer}$ is 0.3 ng/l.

- a noradrenaline expected concentration $[NA]^{expected}$ in one blood sample of said subject and said noradrenaline expected concentration is obtained by applying the following equation

$$[NA]^{expected} = \min(K_1; \left|[NA] - \overline{[NA]_{Alzheimer}}\right|)$$

wherein $K_1$ is a constant comprised between 5 and 25, and
wherein $[NA]_{Alzheimer}$ is 0.8 ng/l.

3. The method according to any one of claims 1 and 2, wherein in step a) at least eight criteria are determined.

4. The method according to any one of claims 1 to 3, wherein in step c) the weighting factor is comprised between 0.1 and 0.9, preferentially equals to 0.5 for the criteria of the age and the score of said subject and comprised between 1.1 and 3.0, preferentially equals to 2, for the criteria of concentrations and the expected concentrations.

5. The method according to any one of claims 1 to 4, wherein said blood sample of said subject is one plasma sample of said subject.

6. The method according to any one of claims 1 to 5, wherein said dopamine, adrenaline and noradrenaline concentrations are determined with an HPLC equipped with an electrochemical detector.

7. The method according to any one of claims 1-6, wherein said method further comprises determining whether said subject suffers from arterial hypertension.

8. The method according to any one of claims 1 to 7, wherein said reference values used in step (b) are as followed:

    a. the age of said subject is comprised within the range 29.9 years and 100.3 years,
    b. the score of said questionnaire adapted for screening cognitive function is comprised between 0 and 30,
    c. the dopamine concentration is comprised between 0.0 ng/ml and 28.2 ng/ml,
    d. the adrenaline concentration is comprised between 0.0 ng/ml and 25.0 ng/ml,
    e. the noradrenaline concentration is comprised between 0.0 ng/ml and 114.0 ng/ml,
    f. the dopamine expected concentration is comprised between 0.0 ng/ml and 10.0 ng/ml,
    g. the adrenaline expected concentration is comprised between 0.0 ng/ml and 10.0 ng/ml,
    h. the noradrenaline expected concentration is comprised between 0.0 ng/ml and 10.0 ng/ml.

9. The method according to any one of claims 1-8, wherein a correction factor $\delta$ is attributed to the reference values, said correction factor $\delta$ being comprised between 0.50 and 1.50.

**Patentansprüche**

1. Ein *In-vitro* -Verfahren zur Durchführung einer Differentialdiagnose neurodegenerativer Erkrankungen bei einem Subjekt, wobei das Subjekt aus Subjekten ausgewählt wird, die an Alzheimer, psychischer Depression, amyotropher Lateralsklerose, frontotemporaler Demenz, Parkinson-Krankheit, progressiver supranukleärer Parese und/oder Parkinson-Krankheit mit Demenz,
wobei das Verfahren die folgenden Schritte umfasst:

    a) Bestimmen von mindestens fünf Kriterien des Subjekts, wobei die mindestens fünf Kriterien Folgendes umfassen: das Alter des Subjekts, eine Bewertung des Subjekts in einem Fragebogen, der zum Screening der kognitiven Funktion angepasst ist, eine Dopaminkonzentration [D] in einer Blutprobe des Subjekts, eine Adrenalinkonzentration [A] in einer Blutprobe des Subjekts, eine Noradrenalinkonzentration [NA] in einer Blutprobe des Subjekts;

    b) eines Werts pro Kriterium $V^{criterion}_{disease}$ für jede neurodegenerative Erkrankung durch Vergleich eines gemessenen Werts $VAL^{criterion}$ der dem erhaltenen Kriterium in Schritt a) entspricht, mit einem Bereich von Referenzwerten wie folgt:

i. $V_{disease}^{criterion} = 0$, wenn VAL^criterion außerhalb des Referenzwertbereichs liegt $\text{MINI}_{disease}^{criterion} - \text{MAXI}_{disease}^{criterion}$, oder

ii. $V_{disease}^{criterion} = 1 + K \dfrac{VAL^{criterion} - MINI_{disease}^{criterion}}{MAXI_{disease}^{criterion} - MINI_{disease}^{criterion}}$ wenn VAL^criterion liegt im Bereich der Referenzwerte

wobei $\text{MINI}_{disease}^{criterion}$ und $\text{MAXI}_{disease}^{criterion}$ jeweils die minimalen und maximalen Werte des Referenzwertbereichs in Bezug auf jede neurodegenerative Erkrankung in Abhängigkeit von jedem Kriterium darstellen,
wobei K ein mathematischer Parameter ist, der zwischen 0 und 0,1 liegt

c) Berechnen einer Gesamtnote für jede neurodegenerative Erkrankung durch Summieren der $V_{disease}^{criterion}$ jeweils mit einem Gewichtungsfaktor multiplizierten Werte;
d) Bestimmen, basierend auf der höchsten globalen Note, die in Schritt c) für alle neurodegenerativen Erkrankungen berechnet wurde, ob das Subjekt an Alzheimer-Krankheit, psychischer Depression, amyotropher Lateralsklerose, frontotemporaler Demenz, Parkinson-Krankheit, progressiver supranukleärer Parese, Parkinson-Krankheit mit Demenz oder mit gemischter Demenz.

**2.** Verfahren nach Anspruch 1, wobei in Schritt (a) die mindestens fünf Kriterien außerdem die folgenden Kriterien umfassen:

- eine erwartete Dopaminkonzentration [D]^expected die in einer Blutprobe des Subjekts erwartet wird, diese erwartete Dopaminkonzentration wird durch Anwendung der folgenden Gleichung ermittelt

$$[D]^{expected} = \min\left(K_1; \left|[D] - \overline{[D]_{Alzheimer}}\right|\right)$$

wobei $K_1$ eine Konstante zwischen 5 und 25 ist, und
wobei $[D]_{Alzheimer}$ 0,4 ng/l beträgt.

- eine erwartete Adrenalinkonzentration [A], die in einer Blutprobe des Subjekts, die erwartete Adrenalinkonzentration wird durch Anwendung der folgenden Gleichung ermittelt

$$[A]^{expected} = \min\left(K_1; \left|[A] - \overline{[A]_{Alzheimer}}\right|\right)$$

wobei $K_1$ eine Konstante zwischen 5 und 25 ist, und
worin $[A]_{Alzheimer}$ beträgt 0,3 ng/l.

- eine erwartete Noradrenalinkonzentration [NA], die in einer Blutprobe des Subjekts erwartet wird, und diese erwartete Noradrenalinkonzentration wird durch Anwendung der folgenden Gleichung ermittelt

$$[NA]^{expected} = \min\left(K_1; \left|[NA] - \overline{[NA]_{Alzheimer}}\right|\right)$$

wobei $K_1$ eine Konstante zwischen 5 und 25 ist, und
worin $[NA]_{Alzheimer}$ beträgt 0,8 ng/l.

**3.** Verfahren nach einem der Ansprüche 1 und 2, wobei in Schritt a) mindestens acht Kriterien ermittelt werden.

**4.** Verfahren nach einem der Ansprüche 1 bis 3, wobei in Schritt c) der Gewichtungsfaktor zwischen 0,1 und 0,9 liegt, vorzugsweise gleich 0,5 für die Kriterien des Alters und der Punktzahl des Probanden ist und zwischen 1,1 und 3,0 liegt. vorzugsweise gleich 2, für die Konzentrationskriterien und die erwarteten Konzentrationen.

**5.** Verfahren nach einem der Ansprüche 1 bis 4, wobei die Blutprobe des Subjekts eine Plasmaprobe des Subjekts ist.

**6.** Verfahren nach einem der Ansprüche 1 bis 5, wobei die Dopamin-, Adrenalin- und Noradrenalinkonzentrationen mit einer HPLC bestimmt werden, die mit einem elektrochemischen Detektor ausgestattet ist.

**7.** Verfahren nach einem der Ansprüche 1-6, wobei das Verfahren weiterhin die Bestimmung umfasst, ob das Subjekt an arterieller Hypertonie leidet.

**8.** Verfahren nach einem der Ansprüche 1 bis 7, wobei die in Schritt (b) verwendeten Referenzwerte wie folgt sind:

a. das Alter des Probanden liegt zwischen 29,9 und 100,3 Jahren,
b. die Punktzahl des für das Screening der kognitiven Funktion angepassten Fragebogens liegt zwischen 0 und 30,
c. die Dopaminkonzentration liegt zwischen 0,0 ng/ml und 28,2 ng/ml,
d. die Adrenalinkonzentration liegt zwischen 0,0 ng/ml und 25,0 ng/ml,
e. die Noradrenalinkonzentration liegt zwischen 0,0 ng/ml und 114,0 ng/ml,
f. die erwartete Dopaminkonzentration liegt zwischen 0,0 ng/ml und 10,0 ng/ml,
g. die erwartete Adrenalinkonzentration liegt zwischen 0,0 ng/ml und 10,0 ng/ml,
h. die erwartete Noradrenalinkonzentration liegt zwischen 0,0 ng/ml und 10,0 ng/ml.

**9.** Verfahren nach einem der Ansprüche 1-8, wobei den Referenzwerten ein Korrekturfaktor $\delta$ zugeordnet wird, wobei der Korrekturfaktor $\delta$ zwischen 0,50 und 1,50 liegt.

## Revendications

**1.** Procédé *in vitro* pour réaliser un diagnostic différentiel de maladies neurodégénératives chez un sujet, ledit sujet étant sélectionné parmi les sujets souffrant de la maladie d'Alzheimer, de dépression mentale, de sclérose latérale amyotrophique, de démence fronto-temporale, de la maladie de Parkinson, de paralysie supra-nucléaire progressive et/ou de la maladie de Parkinson avec démence,
ledit procédé comprenant les étapes suivantes :

a) déterminer au moins cinq critères dudit sujet, lesdits au moins cinq critères comprenant : l'âge dudit sujet, un score dudit sujet à un questionnaire adapté au dépistage des fonctions cognitives, une concentration de dopamine [D] dans un échantillon de sang dudit sujet, une concentration d'adrénaline [A] dans un échantillon de sang dudit sujet, une concentration de noradrénaline [NA] dans un échantillon de sang dudit sujet ;
b) déterminer pour chaque maladie neurodégénérative une valeur $V_{disease}^{criterion}$ par critère en comparant une valeur mesurée VAL$^{criterion}$ correspondant au critère obtenu à l'étape a) avec une plage de valeurs de référence comme suit :

i. $V_{disease}^{criterion}=0$ si VAL$^{criterion}$ est hors de la plage des valeurs de référence $MINI_{disease}^{criterion}$ - $MAXI_{disease}^{criterion}$, ou

ii. $V_{disease}^{criterion} = 1 + K \dfrac{VAL^{criterion} - MINI_{disease}^{criterion}}{MAXI_{disease}^{criterion} - MINI_{disease}^{criterion}}$ si VAL se situe dans la plage des valeurs de référence

dans lequel $MINI_{disease}^{criterion}$ et $MAXI_{disease}^{criterion}$ représentent respectivement les valeurs minimale et maximale de la plage de valeurs de référence par rapport à chaque maladie neurodégénérative en fonction de chaque critère,
dans laquelle K est un paramètre mathématique compris entre 0 et 0,1

c) calculer une note globale pour chaque maladie neurodégénérative en sommant les valeurs $V_{disease}^{criterion}$ multipliées chacune par un facteur de pondération ;
d) déterminer, à partir de la note globale la plus élevée calculée à l'étape c) pour l'ensemble des maladies neurodégénératives, si ledit sujet souffre de la maladie d'Alzheimer, de dépression mentale, de sclérose latérale amyotrophique, de démence fronto-temporale, de maladie de Parkinson, de paralysie supra-nucléaire progressive, de maladie de Parkinson avec démence ou mixte démence.

**2.** Procédé selon la revendication 1, dans lequel à l'étape (a), les au moins cinq critères comprennent en outre les critères suivants :

- une concentration attendue de dopamine [D]$^{expected}$ dans un échantillon de sang dudit sujet, ladite concentration attendue de dopamine est obtenue en appliquant l'équation suivante

$$[D]^{expected} = \min\left(K_1; \left|[D] - \overline{[D]_{Alzheimer}}\right|\right)$$

dans laquelle $K_1$ est une constante comprise entre 5 et 25, et
où [D]$_{Alzheimer}$ est 0,4 ng/l.

- une concentration attendue d'adrénaline [A]$^{expected}$ dans un échantillon de sang dudit sujet ladite concentration attendue d'adrénaline est obtenue en appliquant l'équation suivante

$$[A]^{expected} = \min\left(K_1; \left|[A] - \overline{[A]_{Alzheimer}}\right|\right)$$

dans laquelle $K_1$ est une constante comprise entre 5 et 25, et
où [A]$_{Alzheimer}$ est de 0,3 ng/l.

- une concentration attendue de noradrénaline [NA]$^{expected}$ dans un échantillon de sang dudit sujet et ladite concentration attendue de noradrénaline sont obtenues en appliquant l'équation suivante

$$[NA]^{expected} = \min\left(K_1; \left|[NA] - \overline{[NA]_{Alzheimer}}\right|\right)$$

dans laquelle $K_1$ est une constante comprise entre 5 et 25, et
où [NA]$_{Alzheimer}$ est de 0,8 ng/l.

**3.** Procédé selon l'une quelconque des revendications 1 et 2, dans lequel à l'étape a) au moins huit critères sont déterminés.

**4.** Procédé selon l'une quelconque des revendications 1 à 3, dans lequel à l'étape c) le facteur de pondération est compris entre 0,1 et 0,9, préférentiellement égal à 0,5 pour les critères de l'âge et du score dudit sujet et compris entre 1,1 et 3,0, préférentiellement égal à 2, pour les critères de concentrations et les concentrations attendues.

**5.** Procédé selon l'une quelconque des revendications 1 à 4, dans lequel ledit échantillon de sang dudit sujet est un échantillon de plasma dudit sujet.

**6.** Procédé selon l'une quelconque des revendications 1 à 5, dans lequel lesdites concentrations de dopamine, d'adrénaline et de noradrénaline sont déterminées avec une HPLC équipée d'un détecteur électrochimique.

**7.** Procédé selon l'une quelconque des revendications 1 à 6, dans lequel ledit procédé comprend en outre la détermination si ledit sujet souffre d'hypertension artérielle.

**8.** Procédé selon l'une quelconque des revendications 1 à 7, dans lequel lesdites valeurs de référence utilisées à l'étape (b) sont les suivantes :

a. l'âge dudit sujet est compris entre 29,9 ans et 100,3 ans,
b. le score dudit questionnaire adapté au dépistage des fonctions cognitives est compris entre 0 et 30,
c. la concentration en dopamine est comprise entre 0,0 ng/ml et 28,2 ng/ml,
d. la concentration en adrénaline est comprise entre 0,0 ng/ml et 25,0 ng/ml,
e. la concentration en noradrénaline est comprise entre 0,0 ng/ml et 114,0 ng/ml,
f. la concentration attendue en dopamine est comprise entre 0,0 ng/ml et 10,0 ng/ml,
g. la concentration attendue d'adrénaline est comprise entre 0,0 ng/ml et 10,0 ng/ml,
h. la concentration attendue de noradrénaline est comprise entre 0,0 ng/ml et 10,0 ng/ml.

9. Procédé selon l'une quelconque des revendications 1 à 8, dans lequel un facteur de correction $\delta$ est attribué aux valeurs de référence, ledit facteur de correction $\delta$ étant compris entre 0,50 et 1,50.

**Fig. 1**

| Neurodegenerative disease | Number of patients |
|---|---|
| AD | 30 |
| PSY | 18 |
| ALS | 12 |
| FTD | 15 |
| PD | 8 |
| PSP | 10 |
| PD+ | 4 |
| Control | 105 |
| **TOTAL** | **202** |

OD — brackets ALS, FTD, PD, PSP, PD+

## Fig. 2

## Fig. 3

**Fig. 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013038014 A1

**Non-patent literature cited in the description**

- **SIMIC G. et al.** *Neuropathol. Appl. Neurobiol.,* 2009, vol. 35, 532-554
- **TRILLO L. et al.** *Neurosci Biobehav. Rev.,* 2013, vol. 37, 1363-1379
- **SARA S. J.** *Nat. Rev. Neurosci.,* 2009, vol. 10, 211-223
- **ANTON A. H. et al.** *J. Pharmacol. Exp. Ther.,* 1962, vol. 138 (3), 360-375